# EUROPEAN PATENT SPECIFICATION

(11) **EP 2 495 302 B1**
(45) Date of publication and mention of the grant of the patent: **30.08.2017**
(21) Application number: 10827011.7
(22) Date of filing: 21.10.2010
(51) Int. Cl.: C12M 1/26, C12M 1/24

(54) **BIO-DEVICE FOR EXTRACTING HEMATOPOIETIC STEM CELLS AND MESENCHYMAL STEM CELLS IN PERIPHERAL BLOOD**
BIOVORRICHTUNG ZUR EXTRAKTION BLUTBILDENDER STAMMZELLEN UND MESENCHYMALER STAMMZELLEN IM PERIPHEREN BLUT
BIO-DISPOSITIF D'EXTRACTION DE CELLULES SOUCHES HÉMATOPOÏÉTIQUES ET DE CELLULES SOUCHES MÉSENCHYMATEUSES DANS LE SANG PÉRIPHÉRIQUE

(30) Priority: 29.10.2009 KR 20090103142
(43) Date of publication of application: 05.09.2012
(73) Proprietor: Prodizen, Inc., Nonhyeon-dong, Gangnam-gu Seoul (KR)
(72) Inventor: MOON, Sang Ho, Seoul 152-050 (KR)
(74) Representative: Stevens, Jason Paul
(86) International application number: PCT/KR2010/007238
(87) International publication number: WO 2011/052927

(56) References cited:
- JP-A- 7 140 136
- KR-A- 20080 025 050
- KR-A- 20100 105 282
- KR-B1- 100 917 795
- US-A- 4 861 477

## Description

### BACKGROUND OF THE INVENTION

### FIELD OF THE INVENTION

The following description relates to a bio device for extracting hematopoietic stem cells and mesenchymal stem cells within peripheral blood capable of extracting Buffy coat which is a thin layer included in centrifuged blood.

### DESCRIPTION OF THE RELATED ART

Blood is separated into upper plasma part and lower erythrocyte part when it is centrifuged and a thin layer called as "Buffy coat", consisting of platelets and lymphocytes is located between the plasma part and erythrocyte part. The lymphocyte herein includes non-granulocytes and granulocytes. A part of monocytes among the granulocytes are known as stem cells. In addition, various growth factors secreted in the platelets take part in activating the stem cells.

Such stem cells are used in various fields such as regeneration of cartilage and infarcted muscles as well as plastic surgeries and dermatologic procedures.

However, previous methods for collecting stem cells, extracting blood from a patient by inserting needles to his/her two arms for maximum 5 hours or extracting stem cells from patients' bone marrow directly have disadvantages such as burden of general anesthesia and pain of patients.

In the meantime, there are hematopoietic stem cells (HSCs) and mesenchymal stem cells (MSCs) in peripheral blood in tiny amount and the HSCs and the MSCs are concentrated in Buffy coat layer after centrifugation of blood.

Thus, when a buffy coat layer including stem cells is extracted from a patient's own peripheral blood, and is used for the patient, it is unnecessary to culture stem cells outside the body or in other institutions than hospitals, thereby safely handling the stem cells. In addition, the operation is performed with homogenous peripheral blood of patients thus there is no rejection. Further, because only small amount of blood is needed, pain of patients is alleviated and there is no burden of general anesthesia.

In the related art, typical cylindrical test tubes are used to extract a buffy coat layer. However, since volume of buffy coat included in blood is about 1%, which is a very small amount, it is difficult to identify a buffy coat layer in the test tube. Although buffy coat is identified, it is difficult to extract it separating from erythrocytes.

US 4861477 discloses a tubular container for centrifugal separation of a relatively small amount of the phase having an intermediate specific gravity from the heaviest and lightest phases.

### DISCLOSURE OF THE INVENTION TECHNICAL PROBLEM

The present invention is devised to the problems described above and the purpose of the present invention is to provide bio devices for extracting hematopoietic stem cells (HSCs) and mesenchymal stem cells (MSCs) from peripheral blood, which is capable of extracting and isolating Buffy coat comprising the HSCs and the MSCs.

### TECHNICAL SOLUTION

In a first aspect of the present invention, a bio device for extracting hematopoietic stem cells and mesenchymal stem cells from peripheral blood, comprising
a main body having an upper reservoir, a lower reservoir and a bottleneck part connecting the upper reservoir and the lower reservoir;
a top cover coupled with the upper reservoir of the main body, capable of sliding in the longitudinal direction of the main body; and
a bottom cover coupled with the lower reservoir of the main body, capable of sliding in the longitudinal direction of the main body so as to seal the lower reservoir,
wherein the top cover is equipped with a syringe guide extending downward therefrom, the syringe guide being a tubular shape whose top face and bottom face are penetrated so as that a syringe can be inserted therein and an outlet of the syringe can be positioned inside the main body, and the bottom of the syringe guide can block the bottleneck part and space above the bottleneck part when the syringe guide extends downward is provided.

In this case, the top cover can comprise one or more blood inlet. In addition, the lower part of the upper reservoir can get narrower toward the end and upper part of the lower reservoir can get narrower toward the beginning on the contrary. According to more preferred embodiment, the bio device of the present invention may further comprise one or more plug for sealing the blood inlet.

In addition, the bottom cover can control length of the lower reservoir, since it is screwed or unscrewed to the main body and slides upward or downward thereby.

### ADVANTAGEOUS EFFECTS

The present invention has following effects:
Firstly, one can discriminate Buffy coat layer, a thin layer between plasma and erythrocytes more easily since narrow path of bottleneck part thickens the layer when he or she positioned the layer within the path.
Secondly, it is possible to isolate blood in a particular amount predetermined since one can block the upper reservoir and the lower reservoir separately after centrifugation.
Thirdly, one can aspirate only Buffy coat layer exactly through a syringe inserted to a syringe guide after the Buffy coat is positioned at the bottleneck part and the syringe guide blocks the Buffy coat from plasma in the upper reservoir.

Lastly, one can control volume of the lower reservoir, since the bottom cover is screwed to the main body and he or she can perceive the length to which the bottom cover slides when the bottom cover rotate once.

### BRIEF DESCRIPTION OF DRAWINGS

Fig. 1 is a prospective view of a bio device for extracting hematopoietic stem cells and mesenchymal stem cells from peripheral blood having a blocking column as a stopper.
Fig. 2 is a schematic sectional view for explaining the bio device illustrated in Fig. 1.
Fig. 3 is a prospective view of a bio device 1''' extracting hematopoietic stem cells and mesenchymal stem cells from peripheral blood having a syringe guide 21 as a stopper according to an embodiment of the present invention.
Fig. 4 is a schematic sectional view of the bio device illustrated in Fig. 3.
Fig. 5 is a schematic diagram for explaining a process of extracting Buffy coat using the bio device illustrated in Fig. 2.

### Description of numerical symbols for major parts of drawings

1',1",1''' : a bio device
10 : a main body
11 : an upper reservoir
12 : a lower reservoir
13 : a bottleneck part
14 : a path
15 : a valve
16 : a closing column
20 : a top cover
21, 21' : a syringe guide
21a, 21a' : a syringe hole
22 : a syringe guide hole
23, : a blood inlet
24 : a plug
30 : a bottom cover
31 : a packing part
32 : a supporting part
40 : a syringe
41 : an outlet

### DETAILED DESCRIPTION

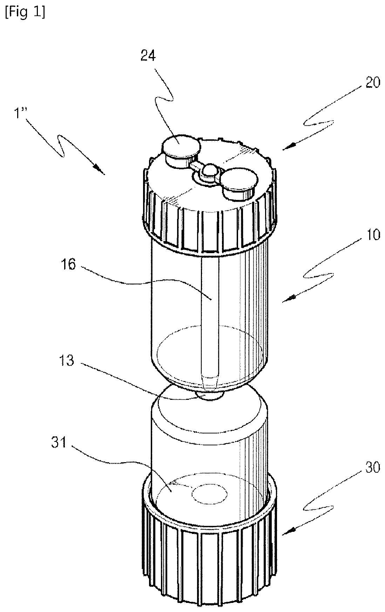

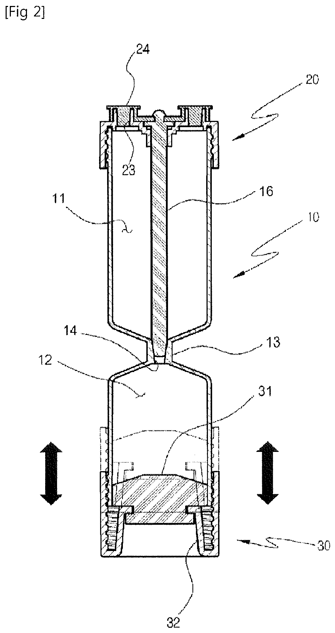

As illustrated in Figs. 1 and 2, the bio device 1'' comprises a main body 10, a top cover 20 and a bottom cover 30.

The main body 10 has tube-like structure and an upper reservoir 11 and a lower reservoir 12 at the upper part and the lower part thereof, respectively. The upper reservoir 11 and the lower reservoir 12 are connected by a bottleneck part 13 which has a narrow path 14. Since the bottleneck part 13 has a relatively smaller volume than other part of the main body part and Buffy coat layer forming border between plasma and erythrocyte layer gets thicker when it is located within the bottleneck part 13, so the border can be seen easily.

In addition, the upper reservoir 11 has a lower part near bottom end, getting narrower and the lower reservoir 12 has a upper part near top end, getting narrower. Since the lower part of the upper reservoir can be narrow toward the end and the upper part of the lower reservoir can be narrow toward the beginning on the contrary, isolated blood may migrate easily through the path 14 of the bottleneck part 13 by controlling a bottom cover 30 described below. In other words, the isolated blood may be squeezed up smoothly through slope of opening of the path 14.

The top cover 20 is a cap covering upper part of main body 10 by being coupled with the main body 10, so as to be capable of sliding in the longitudinal direction of the main body. In this case, because the top cover 20 is screwed to the main body 10, it can repeat in the longitudinal direction of the main body 10 by rotating the same.

In addition, the top cover 20 may further comprise one or more blood inlet 23. In this case, the blood inlet 23 may be a nozzle for injecting blood to be centrifuged. Further, according to more preferred aspect of the present disclosure the blood inlet 23 may be sealed by a plug 24 in order to prevent contamination of blood, but not limited thereto.

The bottom cover 30 is screwed to the bottom of the main body 10 and the lower end of the bottom cover 30 may have a supporting part 32 having hollow structure, which has smaller inner diameter than that of the lower reservoir 12 so as to be inserted thereto. In this case, the upper end of the supporting part 32 may have a packing part 31 for sealing the lower reservoir 12. The outer face of the packing part 31 adheres to the inner face of the lower reservoir 12 when the packing part 31 is inserted to the lower reservoir 12.

The bottom cover 30 equipped with the packing part 31 seals the lower reservoir 12 from the bottom via the packing part 31 and it decreses the volume of the lower reservoir 12 by elevating the packing part 31 accompanying with the elevation of the bottom cover 30 when it is rotated.

On the contrary, if the bottom cover is rotated to the opposite direction, the volume of the lower reservoir 12 decreses according to descending of the packing part 31 accompanying with the descending of the bottom cover 30. That is, the volume of the lower reservoir can be controlled by the bottom cover 30.

As illustrated in Figs. 1 to 3, the upper reservoir 11 and the lower reservoir 12 can be isolated each other completely via the bottleneck part 14. For this, a stopper may be positioned at the bottleneck part 14. The stopper may be a closing column 16 and the closing column may be a syringe guide 21, but not limited thereto.

Fig. 1 is a prospective view of a bio device 1'' for extracting mesenchymal stem cells from peripheral blood, which is equipped with a closing column 16 as a stopper, and Fig. 2 is a schematic sectional view of the bio device illustrated in Fig. 1.
In this case, the closing column 16 is extending downward from the center of the bottom face of the top cover 20. The closing column 16 connected to the top cover 20 can block the path 14 of the bottleneck part 13 by descending when the top cover 20 is rotated clockwise (or counterclockwise if a left-handed screw is used). In this case, one can place the Buffy coat above blocking border by controlling the bottom cover 30.

In addition, the top cover 20 may further comprise one or more blood inlet 23. In this case, the blood inlet 23 may be a nozzle for injecting blood to be centrifuged. Further, according to more preferred aspect of the present disclosure, the blood inlet 23 may be sealed by a plug 24 in order to prevent contamination of blood, but not limited thereto.

The bottom cover 30 is screwed to the bottom of the main body part 10 and the lower end of the bottom cover 30 may have a supporting part 32 having hollow structure, which has smaller inner diameter than that of the lower reservoir 12 so as to be inserted thereto. In this case, the upper end of the supporting part 32 may have a packing part 31 for sealing the lower reservoir 12. The outer face of the packing part 31 adheres to the inner face of the lower reservoir 12 when the packing part 31 is inserted to the lower reservoir 12.

The bottom cover 30 equipped with the packing part 31 seals the lower reservoir 12 from the bottom via the packing part 31 accompanying with the elevation of the bottom cover 30 when it is rotated.

On the contrary, if the bottom cover is rotated to the opposite direction, the volume of the lower reservoir 12 decreases according to descending of the packing part 31 accompanying with the descending of the bottom cover 30. That is, the volume of the lower reservoir 12 can be controlled by the bottom cover 30.

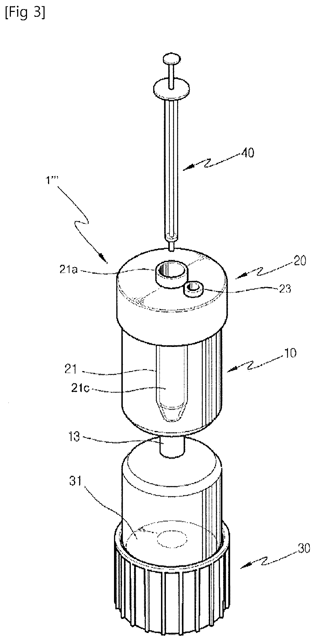

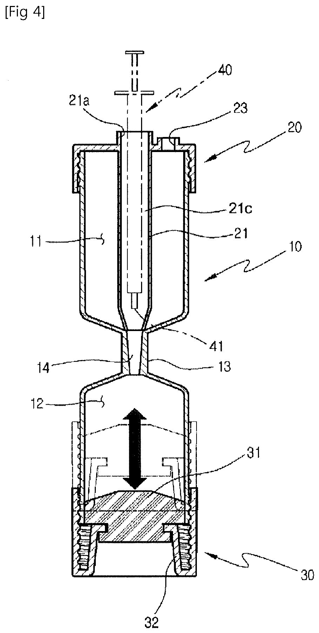

Fig. 3 is a prospective view of a bio device 1*'''* for extracting hematopoietic stem cells and mesenchymal stem cells according to an embodiment of the present invention and Fig. 4 is a schematic sectional view of the bio device illustrated in Fig. 3. In this case, a syringe guide 21 is extending downward from the center of the bottom face of the top cover 20. The syringe guide 21 forms a syringe hole 21a at the center of the top cover 20 where a syringe 41 can be inserted, being a tubular structure having a hollow inside thereby and a blood inlet at the bottom end of the syringe guide 21 is generated so as to protrude an outlet 41 of the syringe 40. The syringe guide 21 is designed so as that the lower part thereof seals the path 14 of the bottleneck part 13, when one lowers the top cover 20 from the main body 10. Also, it is preferred that the lower part of the syringe guide 21 can get narrow toward the bottom end. Therefore, only the outlet 41 of the syringe 40 can protrude below the syringe guide 21 and influx of Buffy coat between the syringe 40 and the syringe guide 21 thereby, when the syringe 40 is inserted to the syringe hole 21a.

In addition, the top cover 20 is a cap covering upper part of main body 10 by being coupled with the main body 10, so as to be capable of sliding in the longitudinal direction of the main body. In this case, because the top cover 20 is screwed to the main body 10, it can repeat in the longitudinal direction of the main body 10 by rotating the same.

In addition, the top cover 20 may further comprise one or more blood inlet 23. In this case the blood inlet 23 may be a nozzle for injecting blood to be centrifuged. Further, it is preferred that the blood inlet fits to an outlet of standard syringe to be connected to various syringes.

Further, the bio device 1*'''* according to an embodiment of the present invention may further comprise a plug 24 for sealing the blood inlet 23 generated in the top cover 20 and may optionally comprise a syringe hole plug (not illustrated) for the upper end of the syringe hole generated in the syringe guide 21.

The bottom cover 30 is screwed to the bottom of the main body part 10 and the lower end of the bottom cover 30 may have a supporting part 32 with hollow structure, which has smaller inner diameter than that of the lower reservoir 12 so as to be inserted thereto. In this case, the upper end of the supporting part 32 may have a packing part 31 for sealing the lower reservoir 12. The outer face of the packing part 31 adheres to the inner face of the lower reservoir 12 when the packing part 31 is inserted thereto.

The bottom cover 30 equipped with the packing part 31 seals the lower reservoir 12 from the bottom via the packing part 31 and it decreases the volume of the lower reservoir 12 by elevating the packing part 31 accompanying with the elevation of the bottom cover 30, when it is rotated clockwise (or counter clockwise if a left-handed screw is used). On the contrary, if the bottom cover is rotated to the opposite direction, the volume of the lower reservoir 12 decreases according to descending of the packing part 31 accompanying with the descending of the bottom cover 30. That is, the volume of the lower reservoir 12 can be controlled by the bottom cover 30.

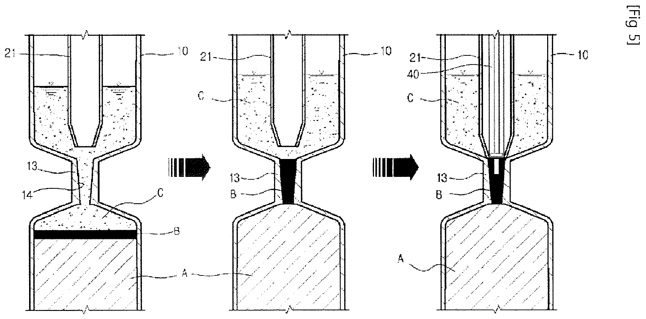

Hereinafter, a process for extracting Buffy coat from peripheral blood by using the bio device 1*'''* according to an embodiment of the present invention is described by referring to Fig. 5.

Firstly, a syringe is filled with an anti-coagulant before sampling blood. Preferable volume of the anti-coagulant is about 10% of volume of blood to be collected. And then, blood of a patient is collected using the syringe filled with the anti-coagulant. Needle of the syringe is removed and inlet of the syringe is sealed to the blood inlet 23 of the top cover 20 in order to prevent leaking of injected blood outside the bio device and the blood is injected into the bio device 1*'''*.

In this, if the blood is injected into the bio device 1*'''* in a condition that the syringe hole 21a of the syringe guide 21 is blocked by a syringe hole plug (not illustrated), the injection of blood may be difficult because the inner space of the main body 10 is airtight. Thus, at least another hole (not illustrated) capable of passing air can be generated in the top cover 20.

However, one may have difficulty to inject blood into the bio device 1*'''* when he or she seals bottom end of the syringe guide 21 to top end of the bottleneck part 13 by rotating the top cover 20 in one direction (clockwise if a right-handed screw is used), because the inner space of the main body 10 is airtight. Further, the injected blood cannot descend to the lower reservoir 12, if the bottleneck part 13 is blocked by the bottom end of the syringe guide 21. Thus, one can easily inject blood to the bio device and the injected blood can descend to the lower reservoir 12 through the bottleneck part 13, whereby he or she loosens the connection between the top cover 20 and the main body 10 and secures space between them by rotating the top cover 20 appropriately in the opposite direction.

Blood is injected into the bio device as the syringe hole 21a is sealed by a syringe hole plug (not illustrated) and then a centrifugation is performed. At this time, penetration of blood or plasma into the syringe guide 21 is prevented due to inner air pressure thereof, because the inside of the syringe guide 21 is sealed with the syringe hole plug.

And then, as the syringe guide 21 is sealed with the syringe hole plug and the bottom end of the syringe guide 21 is spaced apart from the path 14 of the bottleneck part 13, blood inside of the bio device 1*'''* is centrifuged using a centrifuge. After the blood is centrifuged, erythrocytes (A) descend to the lower reservoir 12 of the bio device 1*'''* and plasma (C) is placed above the erythrocytes (A), from the bottleneck part 13 to the upper reservoir 11. Thin Buffy coat layer is located between the plasma (C) and the erythrocytes (A). The reason that the plasma (C) is located above the bottleneck part 13 of the bio device 1*'''* is that the volume of the lower reservoir 12 is set up by operating the bottom cover 30 considering the ratio of plasma (C) in blood. For more precise isolation, the centrifugation may be repeated several times.

And then, the Buffy coat layer (B) is placed in the path 14 of the bottleneck part 13 by controlling the volume of the lower reservoir 12 of the main body 10 by rotating the bottom cover 30. Therefore, thin Buffy coat (B) is accumulated to the path 14 of the bottleneck part 13 which has relatively smaller volume and it is thickened. Thereby, one can discriminate more clearly it from upper plasma (C) and lower erythrocytes (A).

And then, the plasma (C) and Buffy coat (B) are separated by sealing the bottom end of the syringe guide 21 and the top end of the path 14 of the bottleneck part 13 by descending the upper cover 20. Subsequently, the syringe hole plug (not illustrated) blocking the syringe hole 21a of the syringe guide 21 is removed and a syringe 40 is inserted into the syringe hole 21a. Thus, an outlet 41 of the syringe 40 is located on the Buffy coat layer (B). The Buffy coat (B) can be aspired into the syringe 40 when one retreats a piston of the syringe 40 in the state. However, plasma (C) is prevented from moving down to the path 14 of the bottleneck part 13 due to blocking of syringe guide 21, thus he or she can extract only Buffy coat accurately.

Generally, about 0.3 cc of Buffy coat (B) can be extracted when 30 cc of blood is centrifuged. For this end, it is preferable to use 1 cc syringe 40. However, according to standard of a syringe, inner diameter and length of the syringe hole 21a would be determined.

Although the bio-device for extracting hematopoietic stem cells and mesenchymal stem cells from peripheral blood has been described with reference to the specific embodiments, it is not limited thereto. Therefore, it will be readily understood by those skilled in the art that various modifications and changes can be made thereto without departing from the scope of the present invention as defined by the appended claims.

## Claims

1. A bio device (1*'''*) for extracting hematopoietic stem cells and mesenchymal stem cells from peripheral blood, comprising:
a main body (10) having an upper reservoir (11), a lower reservoir (12) and a bottleneck part (13) connecting the upper reservoir and the lower reservoir;
a top cover (20) coupled with the upside of the main body, capable of sliding in the longitudinal direction of the main body; and
a bottom cover (30) coupled with the downside of the main body, capable of sliding in the longitudinal direction of the main body so as to seal the lower reservoir,
wherein the top cover is equipped with a syringe guide (21) extending downward therefrom, the syringe guide being a tubular shape whose top face and bottom face are penetrated so as that a syringe can be inserted therein and an outlet of the syringe can be positioned inside the main body, and the bottom of the syringe guide can block the bottleneck part and space above the bottleneck part when the syringe guide extends downward.

2. The bio device according to claim 1, wherein the top cover further comprises one or more blood inlet (23).

3. The bio device according to claim 1, wherein the bottom cover controls length to which the bottom cover slides, by being screwed to the main body.

4. The bio device according to claim 1, wherein lower part of the upper reservoir gets narrower toward the bottom end thereof and upper part of the lower reservoir gets narrower toward the beginning end.

## Patentansprüche

1. Biovorrichtung (1''*'*) zum Extrahieren hämatopoetischer Stammzellen und mesenchymaler Stammzellen aus peripherem Blut, aufweisend:
einen Hauptkörper (10) mit einem oberen Reservoir (11), einem unteren Reservoir (12) und einem Flaschenhalsteil (13), der das obere Reservoir und das untere Reservoir verbindet;
eine obere Abdeckung (20), die mit der Oberseite des Hauptkörpers gekoppelt ist, die in der Lage ist, in der longitudinalen Richtung des Hauptkörpers zu gleiten; und
eine Bodenabdeckung (30), die mit der Unterseite des Hauptkörpers gekoppelt ist, die in der Lage ist, in der longitudinalen Richtung des Hauptkörpers so zu gleiten, dass das untere Reservoir abgedichtet wird,
wobei die obere Abdeckung mit einer Spritzenführung (21) ausgestattet ist, die sich von dieser nach unten erstreckt, wobei die Spritzenführung eine rohrförmige Gestalt hat, deren obere Oberfläche und Bodenoberfläche so penetriert werden, dass eine Spritze darin eingeführt werden kann, und ein Auslass der Spritze innerhalb des Hauptkörpers positioniert werden kann, und der Boden der Spritzenführung den Flaschenhalsteil und Raum oberhalb des Flaschenhalsteils blockieren kann, wenn sich die Spritzenführung nach unten erstreckt.

2. Biovorrichtung nach Anspruch 1, wobei die obere Abdeckung ferner einen oder mehrere Bluteinlässe (23) aufweist.

3. Biovorrichtung nach Anspruch 1, wobei die Bodenabdeckung durch das Eingeschraubt-sein in den Hauptkörper die Länge steuert, in der die Bodenabdeckung gleitet.

4. Biovorrichtung nach Anspruch 1, wobei der untere Teil des oberen Reservoirs in Richtung des Bodenendes desselben enger wird und der obere Teil des unteren Reservoirs in Richtung des anfänglichen Endes enger wird.

## Revendications

1. Dispositif biologique (1"') pour extraire des cellules souches hématopoïétiques et des cellules souches mésenchymateuses provenant du sang périphérique, comprenant :
un corps principal (10) ayant un réservoir supérieur (11), un réservoir inférieur (12) et une partie formant col (13) reliant le réservoir supérieur et le réservoir inférieur ;
un couvercle supérieur (20) couplé avec le haut du corps principal, susceptible de coulisser dans la direction longitudinale du corps principal ; et
un couvercle inférieur (30) couplé avec le dessous du corps principal, susceptible de coulisser dans la direction longitudinale du corps principal afin de sceller le réservoir inférieur,
dans lequel le couvercle supérieur est équipé d'un guide de seringue (21) s'étendant vers le bas à partir de ce dernier, le guide de seringue ayant une forme tubulaire dont la face supérieure et la face inférieure sont pénétrées de sorte qu'une seringue peut être insérée en son sein et qu'une sortie de la seringue peut être positionnée à l'intérieur du corps principal, et le fond du guide de seringue peut bloquer la partie formant col et l'espace au-dessus de la partie formant col quand le guide de seringue s'étend vers le bas.

2. Dispositif biologique selon la revendication 1, dans lequel le couvercle supérieur comprend en outre une ou plusieurs entrées de sang (23).

3. Dispositif biologique selon la revendication 1, dans lequel le couvercle inférieur commande la longueur de laquelle le couvercle inférieur coulisse, en étant vissé au corps principal.

4. Dispositif biologique selon la revendication 1, dans lequel la partie inférieure du réservoir supérieur devient plus étroite vers l'extrémité inférieure de ce dernier et la partie supérieure du réservoir inférieur devient plus étroite vers l'extrémité de commencement.
